Europäisches Patentamt

European Patent Office

Office européen des brevets

(19)

(11) **EP 0 749 304 B2**

(12) **NEUE EUROPÄISCHE PATENTSCHRIFT**

(45) Veröffentlichungstag und Bekanntmachung des Hinweises auf die Entscheidung über den Einspruch:
**03.03.2004   Patentblatt 2004/10**

(45) Hinweis auf die Patenterteilung:
**18.11.1998   Patentblatt 1998/47**

(21) Anmeldenummer: **95913097.2**

(22) Anmeldetag: **13.03.1995**

(51) Int Cl.⁷: **A61K 31/19**, A61K 9/20

(86) Internationale Anmeldenummer:
**PCT/EP1995/000928**

(87) Internationale Veröffentlichungsnummer:
**WO 1995/024188 (14.09.1995 Gazette 1995/39)**

(54) **RETARDTABLETTE MIT EINEM GEHALT AN DICLOFENAC-NA**

SLOW-RELEASE TABLET CONTAINING DICLOFENAC-NA

COMPRIME A EFFET RETARD CONTENANT DU DICLOFENAC-NA

(84) Benannte Vertragsstaaten:
**AT BE CH DE DK ES FR GB GR IE IT LI LU NL PT SE**

(30) Priorität: **11.03.1994   DE 4408326**

(43) Veröffentlichungstag der Anmeldung:
**27.12.1996   Patentblatt 1996/52**

(73) Patentinhaber: **HEXAL AG**
**D-83607 Holzkirchen (DE)**

(72) Erfinder:
 • **FISCHER, Wilfried**
   **D-83607 Holzkirchen (DE)**
 • **KLOKKERS, Karin**
   **D-83607 Holzkirchen (DE)**

(74) Vertreter: **Boeters, Hans Dietrich, Dr. et al**
**Patentanwälte Boeters & Bauer,**
**Bereiteranger 15**
**81541 München (DE)**

(56) Entgegenhaltungen:
**GB-A- 2 203 338**

 • **Ceska a Slovenska Farmacie 43(1) 22-25**
 • **J. Controlled Release 10, 219-223**
 • **ABPI Datasheet Compendium, 1988-89, p 521**
 • **Pharmazentische Technologie (1978) 333-335**
 • **Methocek - Produktbroschüre 1987 der DOW-Chemical company**

EP 0 749 304 B2

**Beschreibung**

Aufgabe der vorliegenden Erfindung ist es, eine Tablette mit einem Gehalt an Diclofenac-Na vorzusehen, die den Wirkstoff retardierend freisetzt.

Tabletten mit einem Gehalt an Diclofenac-Na als Wirkstoff und Hydroxypropylmethylzellulose als Träger- und Hilfsmittel sind aus WO-A-9 501 781 bekannt. Es ist jedoch erwünscht, Retardtabletten vorzusehen, die den Wirkstoff retardierend freisetzen, so daß auch noch nach einem längeren Zeitraum (> 12 Stunden) eine wirksame Plasmakonzentration an Diclofenac-Na gewährleistet ist.

Diese Aufgabe wird erfindungsgemäß durch eine Retardtablette mit einem Gehalt an Diclofenac-Na als Wirkstoff und Methylhydroxypropylzellulose als Retardierungsmittel sowie üblichen Hilfs-mitteln gelöst, bei der das Verhältnis Methylhydroxy-propylzellulo-se : Didafenac-Natrium ≥ 0,3 beträgt.

Bei in-vitro-Versuchen hat sich gezeigt, daß Tabletten mit einem Gesamtgehalt an 150,0 mg Diclofenac-Na und 35,0 mg Methylhydroxypropylzellulose pro Tablette den Wirkstoff retardierend in etwa acht Stunden zu 100 % freisetzen (Methode: USP XXII, Paddle Apparatur). Unerwarteterweise fand man jedoch, daß sich dieser retardierende Effekt in vivo nicht einstellt, vielmehr nur eine etwa 1-stündige Retardierung erzielen läßt. Es war daher nicht zu erwarten, daß sich durch eine Abänderung des Verhältnisses von Methylhydroxypropylzellulose : Diclofenac-Na eine befriedigende Retardierung in vivo erreichen lassen würde. So läßt sich beispielsweise mit einer erfindungsgemäßen Zweischicht-Retardtablette mit einem Gesamtgehalt an Diclofenac-Na von 150,0 mg Diclofenac-Na pro Tablette und einem Verhältnis von Methylhydroxypropylzellulose : Diclofenac-Na im Retardanteil von 122,5 : 125,0 und einem Verhältnis von Methylhydroxypropylzellulose : Diclofenac-Na im Initialanteil von 0 : 25,0 in vivo eine etwa 15-stündige Retardierung erreichen.

Gemäß einer speziellen Ausführungsform umfaßt eine erfindungsgemäße Retardtablette

(a) einen Tablettenanteil mit einem Gehalt an Diclofenac-Na und Methylhydroxypropylzellulose mit einem Verhältnis von Methylhydroxypropylzellulose : Diclofenac-Na ≥ 0,3 sowie
(b) einen zusätzlichen Tablettenanteil mit einem Gehalt an Diclofenac-Na und Methylhydroxypropylzellulose mit einem Verhältnis von Methylhydroxvpropylzellulose : Diclofenac-Na < 0,3 oder ohne einen Gehalt an Methylhydroxypropylzellulose mit jeweils üblichen Hilfsmitteln,

wobei diese Retardtablette dadurch erhältlich ist, daß man die Tablettenanteile (a) und (b) getrennt voneinander herstellt, danach zusammenbringt und die fertige Retardtablette erhält.

Man kann dazu die Anteile (a) und (b) miteinander verpressen, insbesondere zu einer Mehrschichttablette miteinander verpressen.

Nachstehend wird die Erfindung anhand von Beispielen und Figuren näher erläutert. Es Zeigen:

**Fig. 1** Diclofenac-Na-Freisetzungen in vitro von Tabletten gemäß Beispiel 2, Beispiel 3 oder Vergleichsbeispiel 1;

**Fig. 2** Diclofenac-Na-Freisetzungen in vivo einer Tablette gemäß Beispiel 1 und einer Standard-Tablette des Handels; und

**Fig. 3** Plasmaspiegel einer Tablette gemäß Beispiel 2 und einer Standard-Tablette des Handels;

**Fig. 4** Plasmaspiegel (mit Standardabweichung) einer Tablette gemäß Beispiel 3 nach einmaliger oraler Verabreichung.

**Beispiele 1 und 2:** Retardtablette ohne Initialanteil

| Beispiel | 1 | 2 |
|---|---|---|
| | [mg pro Tablette] | |
| 1 Diclofenac-Natrium | 125,0 | 100,0 |
| 2 Lactose 1$H_2$O | 70,4 | 50,0 |
| 3 Methylhydroxypropylzellulose | 122,5 | 50,0 |
| 4 Farbstoff | 0,1 | 0,0 |
| 5 Wasser zur Granulierung | | |
| 6 Magnesium-Stearat | 3,5 | 2,0 |
| 7 hochdisperses Siliciumdioxid | 3,5 | 0,0 |
| | $\overline{325,0}$ | $\overline{202,0}$ |

Der Farbstoff (4) wurde in Wasser (5) eingerührt Diclofenac-Na (1), Lactose (2) und Methylhydroxypropylzellulose (3) wurden in einem Wirbelschichtgranulator vorgelegt und mit der wässerigen Farbstofflösung granuliert. Das erhaltene Granulat sowie Magnesium-Stearat (6) und hochdisperses Siliciumdioxid (7) wurden duch ein Zwangssieb (1,25 mm) gegeben und in einem Containermischer homogenisiert. Die erhaltene Mischung wurde auf einer Rundlauftablettenmaschine zu Tabletten verpreßt.

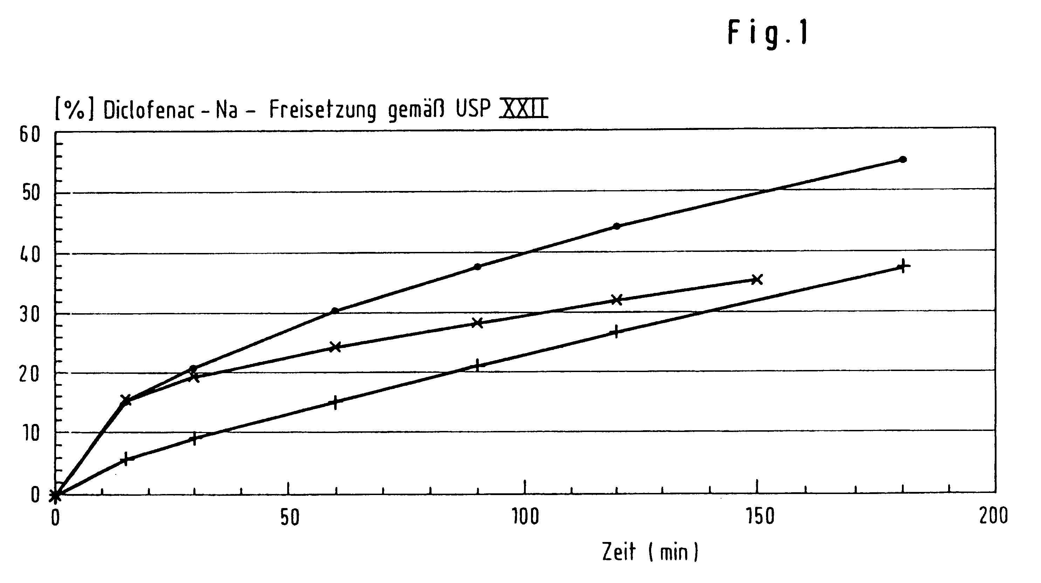

Die Diclofenac-Na-Freisetzung einer Tablette nach Beispiel 2 gemäß USP XXII läßt sich Fig. 1 entnehmen.

**Beispiel 3:** Tablette mit Initial- und Retardanteil (Zweischichttablette)

| | [mg pro Tablette] |
|---|---|
| 1 - 7 wie Beispiel 1 | 325,0 |
| 8 Diclofenac-Natrium | 25,0 |
| 9 Lactose. $1H_2O$ | 15,0 |
| 10 $CaHPO_4$. $2H_2O$ | 20,0 |
| 11 mikrokrist. Zellulose | 24,5 |
| 12 Maisstärke | 10,0 |
| 13 Na-Carboxymethylstärke | 4,0 |
| 14 Magnesium-Stearat | 1,0 |
| 15 hochdisperses Siliciumdioxid | 0,5 |
| | 425,0 |

Beispiel 1 wurde wiederholt. Die vorstehend angegebenen Komponenten (8) bis (15) wurden durch ein Zwangssieb (0,8 mm) gegeben und in einem Containermischer homogenisiert. Die erhaltene Masse wurde als Initialanteil beziehungsweise zweite Schicht auf den gemäß Beispiel 1 erhaltenen Retardanteil zu einer Zweischichttablette aufgepreßt.

Die Diclofenac-Na-Freisetzung gemäß USP XXII läßt sich **Fig. 1** entnehmen.

**Vergleichsbeispiel 1:** Zweischichttablette

| | [mg pro Tablette] |
|---|---|
| 1 Diclofenac-Natrium | 125,0 |
| 2 Lactose. $1H_2O$ | 87,5 |
| 3 Methylhydroxypropylzellulose | 35,0 |
| 4 Farbstoff | 0,0 |
| 5 Wasser zur Granulierung | |
| 6 Magnesium-Stearat | 2,5 |
| 7 hochdisperses Siliziumdioxid | 0,0 |
| 8 Diclofenac-Natrium | 25,0 |
| 9 Lactose. $1H_2O$ | 15,0 |
| 10 $CaHPO_4$. $2H_2O$ | 20,0 |
| 11 mikrokrist. Zellulose | 24,5 |
| 12 Maisstärke | 10,0 |
| 13 Na-Carboxymethylstärke | 4,0 |
| 14 Magnesium-Stearat | 1,0 |
| 15 hochdisperses Siliciumdioxid | 0,5 |
| | 350,0 |

Mit den vorstehend angegebenen Bestandteilen wurde eine Zweischichttablette in Analogie zu Beispiel 3 hergestellt.

**Fig. 1** laßt sich die Diclofenac-Na-Freisetzung gemäß USP XXII als Funktion der Zeit entnehmen.

**Anwendungsbeispiel 1**

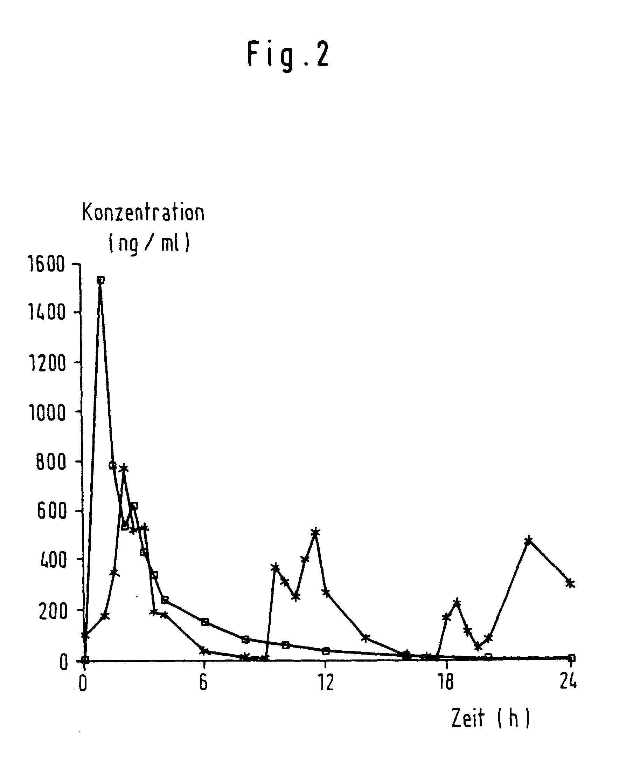

Mit acht Probanden wurde die mittlere Diclofenac-Na-Plasmakonzentration folgendermaßen ermittelt. Und zwar wurde die Konzentration beginnend mit dem vierten Tag nach wiederholter oraler Verabreichung einer Tablette gemäß Beispiel 3 pro Tag (150 mg Diclofenac-Na pro Tag) vier Tage lang verfolgt, wobei jeweils eine Tablette um 8.00 Uhr verabreicht wurde. Der Verlauf der Plasmakonzentration ist in **Fig. 2** durch weiße Quadrate wiedergegeben.

**Anwendungsbeispiel 2**

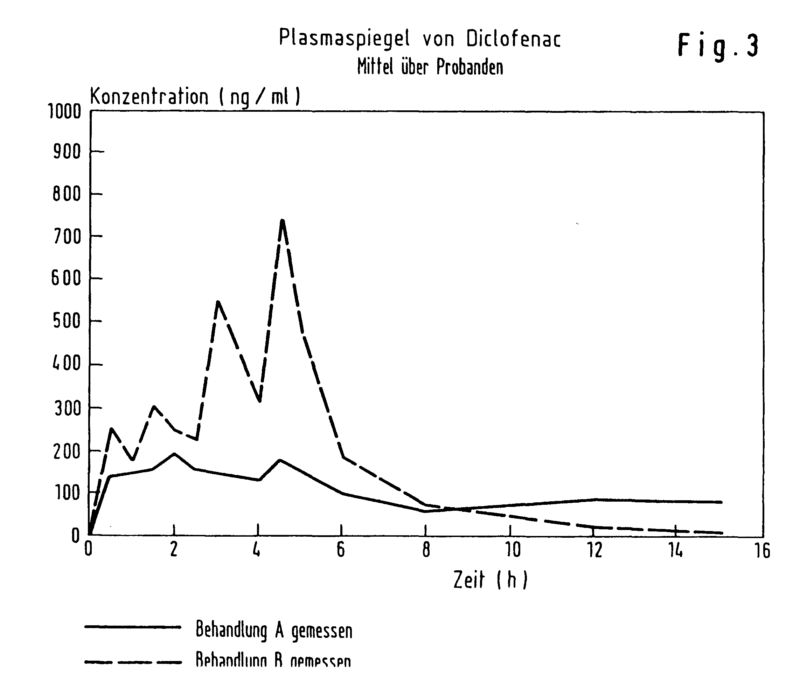

Mit 12 Probanden wurde der mittlere Didofenac-Na-Plasmaspiegel mit einer Tablette gemäß Beispiel 2 ermittelt, wobei der Verlauf in **Fig. 3** mit einer durchgezogenen Linie wiedergegeben ist.

**Vergleichsanwendungsbeispiel 1**

Es wurde Anwendungsbeispiel 1 mit folgenden Ausnahmen wiederholt. Und zwar wurde die mittlere Diclofenac-Na-Konzentration beginnend mit dem vierten Tag nach wiederholter oraler Verabreichung von Diclofenac-Na-Standardtabletten des Handels (50 mg Diclofenac-Na pro Tablette) ermittelt, wobei um 8.00 Uhr, 16.00 Uhr und 0.00 Uhr verabreicht wurde. Der Verlauf der Plasmakonzentration ist in **Fig. 2** durch Sternchen wiedergegeben.

Sowohl beim Anwendungsbeispiel 1 als auch beim Vergleichsanwendungsbeispiel 1 wurden 150 mg Diclofenac-Na pro Tag verabreicht. Ein Vergleich der Plasmakonzentrationen zeigt nun, daß selbst 15 Stunden nach Verabreichung einer erfindungsgemäße Tablette noch eine merklich Plasmakonzentration zu beobachten war, während bei den Vergleichstsbletten die Plasmakonzentration bereits nach etwa 6 Stunden auf einen vergleichbaren Wert abgefallen war.

**Vergleichsanwendungsbeispiel 2**

In diesem Vergleichsanwendungsbeispiel wurde wie in Anwendungsbei spiel 2 der Plasmaspiegel (Mittel über 12 Probanden) einer Diclofenac-Na-Standard-Tablette des Handels (100 mg Diclofenac-Na pro Tablette) ermittelt. Der Verlauf des Plasmaspiegels ist in **Fig. 3** durch eine gestrichelte Linie wiedergegeben.

**Anwendungsbeispiel 3**

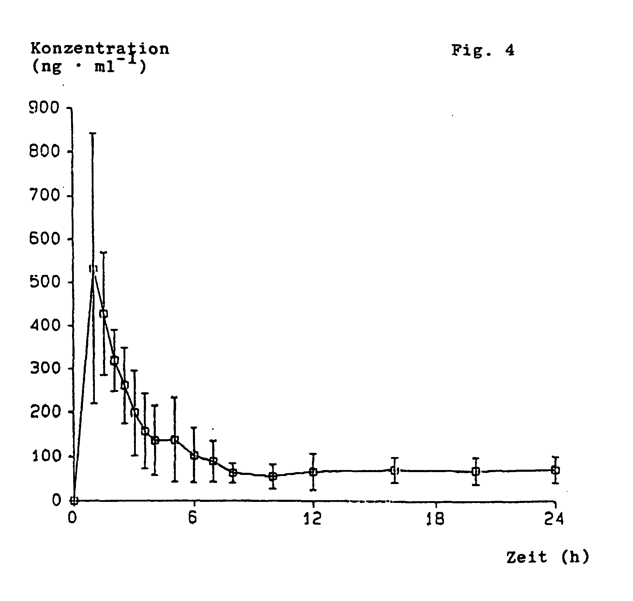

Mit 12 Probanden wurde der mittlere Diclofenac-Na-Plasmaspiegel mit einer Tablette gemäß Beispiel 3 (125,0 mg Initialanteil und 25,0 mg Retardanteil Diclofenac-Natrium) über 24 Stunden verfolgt, wobei der Verlauf in **Fig. 4** durch weiße Quadrate wiedergegeben ist. Die Standardabweichung ist durch senkrechte Balken dargestellt.

**Patentansprüche**

1. Retardtablette mit einem Gehalt an Diclofenac-Na als Wirkstoff und Methylhydroxypropylzellulose als Retardierungsmittel sowie üblichen Hilfsmitteln, **gekennzeichnet durch** ein Verhältnis von Methylhydroxypropylzellulose : Diclofenac-Natrium $\geq 0,3$.

2. Retardtablette nach Anspruch 1, **gekennzeichnet durch**

   (a) einen Tablettenanteil mit einem Gehalt an Diclofenac-Na und Methylhydroxypropylzellulose mit einem Verhältnis von Methylhydroxypropylzellulose : Diclofenac-Na $\geq 0,3$ sowie
   (b) einen zusätzlichen Tablettenanteil mit einem Gehalt an Diclofenac-Na und Methylhydroxypropylzellulose in einem Verhältnis von Methylhydroxypropylzellulose : Diclofenac-Na $\leq 0,3$ oder ohne einen Gehalt an Methylhydroxypropylzellulose

   sowie jeweils üblichen Hilfsmitteln und
   **dadurch** erhältlich, daß man die Tablettenanteile (a) und (b) getrennt voneinander herstellt, danach zusammenbringt und die fertige Retardtablette erhält.

3. Retardtablette nach Anspruch 2, **dadurch gekennzeichnet, daß** man die Anteile (a) und (b) miteinander verpreßt.

4. Retardtablette nach Anspruch 3, **dadurch gekennzeichnet, daß** man die Anteile (a) und (b) miteinander zu einer Mehrschichttablette verpreßt.

### Claims

1. Sustained release tablet with a content of diclofenac Na as active substance and methylhydroxypropyl cellulose as sustained release agent as well as common auxiliary agents, **characterised by** a ratio of methylhydroxypropyl cellulose : diclofenac sodium of $\geq$ 0.3.

2. Sustained release tablet according to claim 1, **characterised by**

   (a) a tablet portion with a content of diclofenac Na and methylhydroxypropyl cellulose with a ratio of methylhydroxypropyl cellulose : diclofenac Na of $\geq$ 0.3 and

   (b) an additional tablet portion with a content of diclofenac Na and methylhydroxypropyl cellulose in a ratio of methylhydroxypropyl cellulose : diclofenac Na of $\leq$ 0.3 or without a content of methylhydroxypropyl cellulose

   and common auxiliary agents respectively and
   obtainable by preparing tablet portions (a) and (b) separately from each other, combining them subsequently and obtaining the finished sustained release tablet.

3. Sustained release tablet according to claim 2 **characterised in that** portions (a) and (b) are compressed together.

4. Sustained release tablet according to claim 3, **characterised in that** portions (a) and (b) are compressed together to form a multiple-layer tablet.

### Revendications

1. Comprimé pour libération prolongée contenant du diclofénac-Na en tant que principe actif et de la méthylhydroxypropylcellulose en tant qu'agent retardateur, ainsi que des adjuvants habituels, **caractérisé en ce que** le rapport méthylhydroxypropylcellulose/diclofénac-Na est supérieur ou égal à 0,3.

2. Comprimé pour libération prolongée selon la revendication 1, **caractérisé par**

   (a) une fraction de comprimé ayant une teneur en diclofenac-Na et une teneur en méthylhydroxypropylcellulose telles que le rapport méthylhydroxypropylcellulose/diclofénac-Na soit supérieur ou égal à 0,3, ainsi que

   (b) une fraction de comprimé supplémentaire ayant une teneur en diclofénac-Na et une teneur en méthylhydroxypropylcellulose telles que le rapport méthylhydroxypropylcellulose/diclofénac-Na soit inférieur ou égal à 0,23 à 0,3, ou une fraction de comprimé exempte de méthylhydroxypropylcellulose,

   ainsi que des adjuvants habituels, et que l'on peut obtenir en préparant séparément les fractions de comprimé (a) et (b) et en les réunissant pour obtenir le comprimé à libération prolongée fini.

3. Comprimé à libération prolongée selon la revendication 2, **caractérisé en ce que** les fractions (a) et (b) sont réunies par compression.

4. Comprimé à libération prolongée selon la revendication 3, **caractérisé en ce que** les fractions (a) et (b) sont réunies par compression en un comprimé multi-couche.

Fig.1

[%] Diclofenac – Na – Freisetzung gemäß USP XXII

Zeit ( min )

# Fig.2

Plasmaspiegel von Diclofenac
Mittel über Probanden

Fig.3

EP 0 749 304 B2

———————— Behandlung A gemessen

— — — — — — Behandlung B gemessen

Fig. 4

Konzentration
$(ng \cdot ml^{-1})$

Zeit (h)